# EUROPÄISCHE PATENTSCHRIFT

(11) **EP 1 438 033 B1**
(45) Veröffentlichungstag und Bekanntmachung des Hinweises auf die Patenterteilung: **11.06.2008**
(21) Anmeldenummer: 02767040.5
(22) Anmeldetag: 22.10.2002
(51) Int. Cl.: A61K 9/48, A61J 3/07

(54) **HARTKAPSELN AUF BASIS VON STÄRKE-GEL UND VERFAHREN ZU DEREN HERSTELLUNG**
HARD CAPSULES BASED ON STARCH GEL AND METHOD FOR THE PRODUCTION THEREOF
CAPSULES DURES A BASE DE GEL D'AMIDON ET PROCEDE PERMETTANT DE LES UTILISER

(30) Priorität: 23.10.2001 DE 10152125; 06.05.2002 DE 10220212
(43) Veröffentlichungstag der Anmeldung: 21.07.2004
(73) Patentinhaber: InnoGEL AG, 6331 Hünenberg (CH)
(72) Erfinder: MÜLLER, Rolf, CH-8055 Zürich (CH)
(74) Vertreter: Frommhold, Joachim
(86) Internationale Anmeldenummer: PCT/CH2002/000576
(87) Internationale Veröffentlichungsnummer: WO 2003/035045

(56) Entgegenhaltungen:
- EP-A- 1 262 174
- WO-A-00/18835
- WO-A-92/04408
- DE-A- 10 022 095
- DE-A- 19 852 826
- US-A- 3 784 390
- US-A- 4 306 059

## Beschreibung

Die vorliegende Erfindung betrifft die Herstellung von Formkörpern im Tauchverfahren auf Basis von Polysaccharid-Gelen, insbesondere von Stärke-Gelen.

Entsprechend dem Stand der Technik werden Formkörper im Tauchverfahren, insbesondere Hartkapseln, heute noch vorwiegend auf Basis von Gelatine hergestellt. Ein Ersatz von Gelatine für diese Anwendung ist aufgrund der BSE-Problematik jedoch dringlich geworden. Weitere Nachteile der Gelatine sind deren tierischer Ursprung und daher die Nicht-Akzeptanz von Gelatine durch Vegetarier, Veganer, Juden (nicht kosher) und Mohammedaner (Schweine-Gelatine).

### Stand der Technik

In US 1787777, US 3032700, US 3802272, US 4026986, US 4196564, US 4250007, US 4268265, US 4576284, US 4738817, US 6000298 werden zweiteilige medizinische Hartkapseln und deren Herstellung auf Basis von Gelatine und Kollagen beschrieben. Solche Lösungen auf Basis tierischer Stoffe sind jedoch aufgrund der erwähnten Punkte problematisch.

In US 4738818, US 4738724, US 4539060, US 4591475, US 4591475 werden Hartkapseln bestehend aus amorpher Thermoplastischer Stärke, hergestellt im Spritzgussverfahren, beschrieben. Die Kapseln sind jedoch spröde und brechen unter den hohen Beanspruchungen in den Hochgeschwindigkeitsabfüllautomaten, sodass sich diese Technologie nicht durchsetzen konnte.

In US 5698155 und US 5431917 werden Hartkapseln auf Basis von wasserlöslichen Cellulosederivaten beschrieben. Diese Hartkapseln zeigen zwar, abgesehen von der problematischen Abfüllung (reduzierte Abfüllgeschwindigkeit), geeignete Eigenschaften, doch sind sie aufgrund der teuren Cellulosederivate nur beschränkt konkurrenzfähig und werden alternative günstigere Lösungen daher gute Chancen auf dem Markt haben.

### Erfindung

Die vorliegende Erfindung stellt sich die Aufgabe, auf Basis von Stärke und anderen Polysacchariden Gelatine-freie Hartkapseln bereitzustellen, die analog zu Gelatine-Hartkapseln im Tauchverfahren hergestellt werden können.

Die Aufgabe ist mit den Merkmalen des Patentanspruchs 1 gelöst. Gegenstand der Erfindung ist ein Verfahren zur Herstellung von Formkörpern gemäss den Ansprüchen 1-6, sowie gemäss diesem Verfahren hergestellte Hartkapseln gemäss den Ansprüchen 7-13.

Die grundlegende Herausforderung besteht darin, hierzu eine viskose Lösung von Polysacchariden herzustellen, die im Temperaturbereich von etwa 30°C bis höchstens 70°C über einen längeren Zeitraum von mindestens einer Stunde stabil bleibt und nachdem eine Negativ-Form, beispielsweise ein Stift, durch Eintauchen in die viskose Lösung und Herausziehen mit dieser Lösung beschichtet worden ist, die Lösung sich durch einen Gelierungsvorgang verfestigt, ausgelöst durch ein Absinken der Temperatur des Films von der Temperatur der Lösung auf die tiefere Temperatur der Negativ-Form, welche üblicherweise mit der Raumtemperatur identisch ist.

Die Anforderung bezüglich der Langzeitstabilität der Lösung ist durch den Stand der Technik des Tauchverfahrens gegeben. Die Grenzen des Temperaturbereichs sind einerseits dadurch gegeben, dass bei zu hoher Temperatur der Lösung die Viskosität und die Konzentration der Lösung infolge von abdampfendem Lösungsmittel in Funktion der Zeit variiert, beziehungsweise zunimmt und daher die mittels des Tauchverfahrens erhaltenen Formkörper nicht von gleichbleibender Qualität sind (variierende Wandstärken, variierendes Herausziehverhalten). Bei der traditionellen Herstellung von Gelatine-Hartkapseln sind diese Effekte relativ klein und korrigierbar, da die Temperatur der Gelatine-Lösung nur wenig mehr als 40°C beträgt und daher die Lösungsmittelverluste gering sind. Andererseits ist die untere Grenze der Lösungs-Temperatur dadurch gegeben, dass gegenüber der Raumtemperatur ein genügend grosser Temperaturgradient vorhanden sein muss, um die Gelierung in möglichst kurzer Zeit auslösen zu können. Dauert die Gelierung zu lange, so fliesst die an der Negativ-Form nach dem Eintauchen und Herausziehen haftende Lösung gravitationsbedingt, und der aus der Lösung nach einer Konditionierung erhaltene Film zeigt untolerierbare Gradienten der Dicke. Ideal ist daher ein Gelierungsvorgang, der sogleich nach dem Herausziehen einsetzt und somit die erhaltene Form fixiert wird. Bei der Konditionierung wird ein Grossteil des Weichmachers oder Lösungsmittels, vorwiegend Wasser aus dem Film entfernt.

Auch ohne Abdampfverluste von Lösungsmitteln sind viele Polysaccharid-Lösungen nicht stabil. Von Stärke beispielsweise ist bekannt, dass Lösungen bei konstanter Konzentration teilweise grosse Veränderungen der Viskosität aufweisen. Der wichtigste Grund hierfür ist durch die Retrogradation von Stärke-Lösungen gegeben. Dieser Effekt ist bei höherer Temperatur weniger ausgeprägt, doch sind dann die Abdampfverluste umso grösser. Das Problem wird andererseits noch dadurch verschärft, dass Stärken, die eine Gelierung ermöglichen, ein besonders ausgeprägtes Retrogradationsverhalten zeigen, insbesondere bei tieferen Temperaturen, wodurch die Bedingung einer über eine längere Zeit stabilen Lösung bei nicht zu hoher Temperatur und die Bedingung einer möglichst raschen Gelierung nach der Formgebung gegenläufig sind, sich zu widersprechen scheinen.

Zur Lösung dieses Problems wurden Zweikomponentensysteme entwickelt, wobei die zwei Komponenten, vorliegende Stärke und netzwerkfähige Polysaccharide, getrennt aufbereitet, d.h. unter spezifischen Bedingungen gelöst und anschliessend miteinander gemischt werden. Essentiell sind dabei weniger die verwendeten Stärken beziehungsweise Polysaccharide, als vielmehr die Parameter der Aufbereitung. Als vorliegende Stärke kann dabei im Prinzip irgendeine Stärke eingesetzt werden, bevorzugt werden jedoch Stärken mit einem Amylosegehalt von weniger als 35%. Um stabile Lösungen solcher Stärken zu erhalten, wurde gefunden, dass eine hohe Lösungstemperatur, insbesondere bei gleichzeitiger Anwendung von hohen Schergeschwindigkeiten, sich sehr vorteilhaft auswirkt. Nach dem Lösen bei genügend hoher Temperatur können die Lösungen gekühlt werden und sind sie dann bezüglich der Retrogradation stabiler als bei niederer Lösungstemperatur. Da diese Stärken jedoch die erwünschten Gelierungseigenschaften nicht aufweisen, wurden weitere Polysaccharide in Betracht gezogen, welche diesen Effekt im geforderten Temperaturbereich ermöglichen, ohne die Stabilität der Lösung zu beeinträchtigen. Diesbezüglich wurden folgende drei Möglichkeiten gefunden:
1) Als gelierende oder netzwerkbildende Stärken wurden eine Reihe von Stärken gefunden, welche als netzwerkfähige Stärken bezeichnet werden. Diese Stärken ermöglichen zwar, nachdem sie in eine Lösung überführt und mit vorliegenden Stärken gemischt wurden, eine Gelierung des Films auf dem Negativ-Formkörper, jedoch können keine stabile Lösungen erhalten werden, da diese Stärken ein ausgeprägtes Retrogradationsverhalten zeigen. Um eine Retrogradation zu vermeiden, müssen unpraktikabel hohe Lösungstemperaturen von mindestens 80°C eingestellt werden. Dieses Problem konnte jedoch gelöst werden, indem die netzwerkfähigen Stärken separat von den vorliegenden Stärken gelöst wurden und bei deutlich höherer Temperatur als zur Herstellung einer Lösung üblich und gemeinhin als notwendig erachtet wird. Es wurde gefunden, dass eine Überhitzung der Lösung von netzwerkfähiger Stärke diese Lösung bei tieferen Temperaturen erstaunlicherweise stabilisiert. Durch eine deutliche Überhitzung von rund 40°C, insbesondere bei der gleichzeitigen Anwendung von hohen Schergeschwindigkeiten, konnte die Temperatur des Retrogradations- oder Gelierungsbeginns um bis zu 40°C zu tieferen Temperaturen abgesenkt werden. Nachdem die überhitzte und anschliessend gekühlte Lösung von netzwerkfähiger Stärke mit der ebenfalls stabilisierten Lösung von vorliegender Stärke gemischt worden ist, sinkt die Konzentration netzwerkfähiger Stärke durch die Verdünnung mit vorliegender Stärke und wird dadurch eine weitere Stabilisierung erhalten, d.h. die Retrogradations- oder Gelierungstemperatur zusätzlich zu tieferen Temperaturen hin verschoben. Durch diese Vorgehensweise gelingt es, im geforderten Temperaturbereich von 40°C bis 70°C hinreichend stabile Lösungen zu erhalten, die vergleichbar mit Gelatine für das Tauchverfahren verwendet werden können. Nach der Beschichtung der Eintauchkörper verfestigt sich der daran haftende Film innerhalb von Minuten durch Gelierung, sodass die erhaltene Form fixiert bleibt. Die getroffenen Verfahrensmassnahmen ermöglichen somit die Herstellung von Hartkapseln auf Basis von Stärke-Gel unter industriellen Produktionsbedingungen mit gleichbleibender Qualität. Um einen thermischen Abbau von netzwerkfähiger Stärke bei den hohen Überhitzungstemperaturen zu unterdrücken, wurde diese Temperatur nur für eine kurze Zeit gehalten. Als besonders vorteilhaft wurden Mischungen von vorliegenden Stärken und netzwerkfähigen Stärken gefunden, die durch Heterokristallisation Netzwerke bilden können, wobei vorliegende Stärken, die entsprechend dem Stand der Technik keine Gel-Bildung zeigen (oder nur sehr schwache Gele bilden bei Gelierungszeiten von Tagen bis Wochen, während für die vorliegende Erfindung Gelierungszeiten von Minuten gefordert werden), nach vorzugsweise molekulardisperser Mischung mit netzwerkfähigen Stärken an der Netzwerkbildung beteiligt sind. Dieser Effekt wurde mit Mischungen von gelöstem Waxy-Mais, der nicht geliert, mit gelösten entzweigten Maltodextrinen, die ebenfalls nicht gelieren, bestätigt, indem daraus unter geeigneten Bedingungen Gele mit den geforderten Gelierungseigenschaften erhalten werden konnten.
2) Als gelierende Komponente wurden weitere gelierende nicht-Stärke-Polysaccharide eingesetzt wie beispielsweise Carrageenane, Alginate, Gellane oder Agar. Auch mit diesen Polysacchariden konnten stabile Lösungen im geforderten Temperaturbereich erhalten werden, indem die Polysaccharide in Lösung kurzzeitig überhitzt und anschliessend mit der Lösung von vorliegenden Stärken gemischt wurden. Da diese Polysaccharide im Vergleich mit netzwerkfähiger Stärke im Allgemeinen bei tieferen Temperaturen gelieren, sind in den meisten Fällen weniger hohe Überhitzungstemperaturen zur Stabilisierung der Lösungen mit vorliegender Stärke notwendig. Ausserdem ist in diesen Fällen auch ein gemeinsamer Lösungsvorgang von vorliegender Stärke mit diesen Polysacchariden möglich, allerdings wird die separate Aufarbeitung bevorzugt.
3) Als gelierende Komponente wurden weiterhin Mischungen von netzwerkfähigen Stärken mit gelierenden nicht-Stärke-Polysacchariden eingesetzt, wobei die Komponenten bevorzugt individuell gelöst und überhitzt und dann mit der Lösung von vorliegender Stärke gemischt wurden.

Für die vorliegende Erfindung bezüglich reiner Stärke-Gele ist von Bedeutung, dass die thermodynamisch begünstigte Separation von amylopektinartigen Stärken (vorliegende Stärke) und amyloseartigen Stärken (netzwerkfähige Stärke) in zwei Phasen durch verschiedene Verfahrensmassnahmen unterdrückt wird, sodass nicht klassische zweiphasige Gele, sondern homogene einphasige Gele entstehen, wobei Amylopektin-Amylose Wechselwirkungen strukturbildend ausschlaggebend sind (Heterokristallisation). Daraus ergeben sich Eigenschaften, die für die Herstellung von Hartkapseln vorteilhaft sind. Die Herstellung solcher einphasiger Gele ist in hohem Masse von den Parametern des Verfahrens abhängig. Die erfindungsgemässen Stärke-Gele sind jedoch nicht völlig homogen und einphasig, es können, abhängig von den Verfahrensparametern auch Bereiche von verschiedenen Ordnungsstrukturen vorliegen. Im Vergleich mit klassischen zweiphasigen Stärke-Gelen, deren Phasen typischerweise grösser sind als 500nm, sind die verschiedenen Ordnungsstrukturen bei einphasigen Stärke-Gelen jedoch typischerweise kleiner als 500nm, sodass diese Gele im allgemeinen transparent sind. Entscheidend für die Gel-Bildung von einphasigen Stärke-Gelen ist, dass Amylose-Amylopektin Wechselwirkungen wesentlich an der Gel-Bildung beteiligt sind, und dass die üblicherweise auftretende Entmischung von Amylose-Amylopektin Mischungen ganz oder teilweise unterdrückt wird.

Erfindungsgemäss werden neben Hartkapseln, die aus einphasigen Stärke-Gelen bestehen auch Hartkapseln beansprucht, die aus zwei oder mehrphasigen Gelen bestehen und entsprechend dem vorgeschlagenen Verfahren hergestellt werden. Allerdings zeigen die einphasigen Stärke-Gele für diese Anwendung günstigere Eigenschaften und werden bevorzugt.

Das Verfahren kann vereinfacht dadurch charakterisiert werden, dass eine erste Lösung von vorliegender Stärke oder eine Lösung einer Mischung von vorliegenden Stärken mit vergleichsweise niederem Weichmacher- oder Lösungsmittelgehalt durch Überhitzung bezüglich der Retrogradation zu tiefen Temperaturen hin stabilisiert, mit einer ebenfalls durch Überhitzung stabilisierten zweiten Lösung von netzwerkfähigen Polysacchariden oder zweiten Lösungen von netzwerkfähigen Polysacchariden (wobei die für die Herstellung von zweiten Lösungen angewandte Überhitzung deutlich ausgeprägter ist als im Falle der ersten Lösung) mit vergleichsweise hohem Weichmacher- oder Lösungsmittelgehalt vorzugsweise molekulardispers gemischt wird, um im Temperaturbereich von 40°C bis 70°C, insbesondere von 45°C bis 60°C, stabile Lösungen zu erhalten, die bei Temperaturen um 20°C innerhalb von Minuten sich durch Gelierung verfestigen und dadurch formstabil bleiben, in diesem Zustand der nachfolgenden Konditionierung zugeführt werden können, wodurch Formkörper, insbesondere Hartkapseln mit gleichbleibender Qualität erhalten werden können, die analog Gelatine-Hartkapseln weiterverarbeitet und verwendet werden können.

### Vorliegende Stärke

Als vorliegende Stärke können im Prinzip irgendwelche Stärken, wie auch Mischungen verschiedener Stärken eingesetzt werden. Bevorzugt werden jedoch Stärken mit einem Amylosegehalt von weniger als 35%. Die vorliegende Stärke kann in einem beliebigen Zustand, physikalisch und/oder chemisch modifiziert dem Verfahren zugeführt werden.

Beispiele für in Frage kommende vorliegende Stärken oder Mehle sind folgenden Ursprungs: Getreide wie Mais, Reis, Weizen, Roggen, Gerste, Hirse, Hafer, Dinkel etc.; Wurzeln und Knollen wie Kartoffel, Süsskartoffel, Tapioka (Cassava), Maranta (Arrowroot) etc.; Hülsenfrüchte und Samen wie Bohnen, Erbsen, Mungo, Lotus etc.. Daneben kommen auch Stärken und Mehle anderen Ursprungs in Frage wie beispielsweise Sago, Yams etc.. Ausserdem kann auch Glycogen eingesetzt werden.

Die Stärken können durch Züchtung oder gentechnische Methoden verändert worden sein wie beispielsweise Waxy-Mais, Waxy-Reis, Waxy-Kartoffel, hochamylosehaltiger Mais, indica Reis, japonica Reis et., sie können durch chemische Verfahren verändert worden sein wie beispielsweise durch Säure-Konvertierung, Pyrokonvertierung, Vernetzung, Acetylierung, Hydroxyethylierung, Hydroxypropylierung, Phosphorylierung, Graft-Reaktionen, Reaktionen mit Amylasen et., sie können durch physikalische Verfahren verändert worden sein wie beispielsweise durch Gelatinisierung (teilweise bis vollständig), Plastifizierung, Inhibierung et., oder sie können durch eine Kombination von Züchtung, genetische Methoden, chemische und physikalische Verfahren verändert worden sein.

Beispiele für veränderte Stärken sind dünnkochende Stärken, kaltwasserlösliche Stärken, pregelatinisierte Stärken, hydroxypropylierte Stärken, Dextrine, Maltodextrine, limit-Dextrine, Oligosaccharide, kationische Stärken, Stärkeether, durch Fraktionierung erhaltene Stärken.

Von besonderem Interesse sind vorliegende Stärken, deren Amylopektin-Fraktion eine mittlere Kettenlänge CL von mindestens 20, vorzugsweise von mindestens 22, noch bevorzugter von mindestens 24, am bevorzugtesten von mindestens 26 aufweisen.

Weiter sind von besonderem Interesse vorliegende Stärken, deren Amylopektin-Fraktion einen Blue-Value (BV) von mindestens 0.10, vorzugsweise von mindestens 0.13, noch bevorzugter von mindestens 0.16, am bevorzugtesten von mindestens 0.18 aufweisen.

Ebenfalls sind von besonderem Interesse vorliegende Stärken, deren Amylopektin-Fraktion eine Jod-Affinität (IA) in g/100g von mindestens 0.4, vorzugsweise von mindestens 0.6, noch bevorzugter von mindestens 0.8, am bevorzugtesten von mindestens 1.0 aufweisen.

Von besonderer Bedeutung für die vorgeschlagene Erfindung sind ausserdem viskositätsstabilisierte Stärken, inhibierte Stärken, vernetzte Stärken oder Novation Stärken (National Starch).

Bezüglich des Molekulargewichts M_{w} (Gewichtsmittel) von vorliegenden Stärken sind von besonderem Interesse Stärken mit einem Gewichtsmittel von mehr als 10'000g/mol, vorzugsweise von mehr als 50'000g/mol, noch bevorzugter von mehr als 100'000g/mol.

### Netzwerkfähige Polysaccharide

Netzwerkfähige Polysaccharide können Polysaccharid-Hydrokolloide wie beispielsweise Stärken, Agar, Agarosen, Carrageenane, Alginate, Gellane oder Pektine sein, sie können nativ oder physikalisch und/oder chemisch modifiziert worden sein und lassen sich auf folgende Arten definieren:
1. Entsprechend einer ersten Definition können diese Polysaccharide, insbesondere Stärken sein, die unter geeigneten Bedingungen Gele bilden können. Davon ausgenommen sind Gele wie reine Amylopektin-Gele, die sehr lange Gelierungszeiten (Tage bis Wochen) benötigen und dann nur sehr schwache Gele bilden.
1A. Eine Gruppe von Stärken, die dieser Anforderung genügen, sind native oder modifizierte Stärken mit einem Amylosegehalt von mindestens 10%, vorzugsweise von mindestens 20%, noch bevorzugter von mindestens 30%, am bevorzugtesten von mindestens 50%. Besonders geeignet sind beispielsweise hochamylosehaltige Stärken, insbesondere hochamylosehaltige Maisstärken, die einen Amylosegehalt bis nahezu 100% aufweisen können, Erbsenstärken mit Amylosegehalten von mehr als 25% oder Amylosen beliebigen Ursprungs.
1B. Eine weitere Gruppe von netzwerkfähigen Polysacchariden, kann durch chemischen und/oder enzymatischen Abbau, insbesondere durch Entzweigung erhalten werden. Für den enzymatischen Abbau von Stärken beispielsweise können Amylasen, wie alpha-Amylase, beta-Amylase, Glucoamylase, alpha-Glucosidase, exoalpha-Glucanase, Cyclomaltodextrin, Glucanotransferase, Pullulanase, Isoamylase, Amylo-1,6-Glucosidase oder eine Kombination dieser Amylasen eingesetzt werden. Als Ausgangsstoffe für den Abbau werden dabei insbesondere Stärken der vorgenannten Gruppe von Stärken eingesetzt. Ein Beispiel von chemischem, nicht-enzymatischem Abbau von Polysacchariden, ist die Hydrolyse mittels Säuren wie etwa Salzsäure.
2. Eine Definition netzwerkfähiger Stärken bezieht sich auf den Verzweigungsgrad Q_{b} der netzwerkfähigen Stärken, wobei der Verzweigungsgrad im Bereich 0.02 - 0.001, vorzugsweise im Bereich als 0.01 - 0.001, noch bevorzugter im Bereich 0.007 - 0.001 liegt, wobei der mittlere Polymerisationsgrad DP grösser ist als 100, vorzugsweise grösser als 200, am bevorzugtesten grösser als 300.
3. Eine Definition netzwerkfähiger Polysaccharide bezieht sich auf auf den Verzweigungsgrad Q_{b} der netzwerkfähigen Polysaccharide wobei der Verzweigungsgrad kleiner ist als 0.02, vorzugsweise kleiner als 0.01, noch bevorzugter kleiner als 0.007, insbesondere kleiner als 0.003, am bevorzugtesten kleiner als 0.001.
4. Ausserdem werden als netzwerkfähige Polysaccharide auch vorwiegend lineare Polysaccharide bezeichnet, die nach erfolgter Lösung kristallisieren können, in Abwesenheit weiterer Polysaccharide jedoch keine Gele, sondern Dispersionen von Kristalliten bilden. Solche Polysaccharide haben mittlere Polymerisationsgrade DP von typischerweise weniger als 100, sie können jedoch in Anwesenheit von Polysacchariden, die sowohl nicht netzwerkfähig, als auch netzwerkfähig sein können, durch Heterokristallisation unter geeigneten Bedingungen Gele bilden. Bezüglich dieses Typs von netzwerkfähigen Polysacchariden sind Polysaccharide, insbesondere Stärken von Interesse, die eine mittlere Kettenlänge CL oder einen mittleren Polymerisationsgrad von mindestens 10, vorzugsweise von mindestens 20, noch bevorzugter von mindestens 30, am bevorzugtesten von mindestens 50 aufweisen. Im Falle von Stärken kann eine solche netzwerkfähige Stärke beispielsweise ein entzweigtes Maltodextrin sein, das für sich keine Gele bilden kann, jedoch mit einem Amylopektin Gele bildet, die Amylose-Gelen vergleichbar sind.
5. Netzwerkfähige Polysaccharide können andererseits dadurch charakterisiert werden, dass die Makromoleküle lineare Anteile enthalten, wobei diese linearen Anteile Haupt- oder Seitenketten sein können mit mittleren Polymerisationsgraden DP von mehr als 30, vorzugsweise mehr als 50, am bevorzugtesten mehr als 80, insbesondere von mehr als 100, am insbesondersten von mehr als 140.
6. Ausserdem kann eine weitere Gruppe von netzwerkfähigen Stärken durch Fraktionierung von Amylose-Amylopektin-Mischungen erhalten werden, beispielsweise durch Fraktionierung mittels differentieller Alkoholfällung, wobei die Amylose- und die intermediate Fraktion als netzwerkfähige Stärke eingesetzt werden kann.

Erfindungsgemäss werden als netzwerkfähiges Polysaccharid Polysaccharide bezeichnet, welche mindestens eine der Bedingungen 1 - 6 erfüllen. Als netzwerkfähiges Polysaccharid werden auch Mischungen bezeichnet, wobei die Komponenten und/oder die Mischung mindestens eine der obigen Bedingungen erfüllen.

Es wird darauf hingewiesen, dass in bestimmten Fällen vorliegende Stärke und netzwerkfähige Stärke stofflich identisch sein können. Der Unterschied zwischen vorliegender Stärke und netzwerkfähiger Stärke ist daher nicht in allen Fällen stofflicher Art, vielmehr müssen die Begriffe auch in Zusammenhang mit dem Verfahren definiert werden. Netzwerkfähige Stärken werden in einer Weise behandelt, dass deren Potential zur Bildung von Netzwerken optimal freigesetzt wird, während dies bei vorliegenden Stärken nicht der Fall sein muss.

### Verfahren

### 1. Lösen und Kühlen vorliegender Stärke

1. Der Weichmachergehalt WM_{c} in Gew.% der vorliegenden Stärken in Schritt c) vor dem Zuführen der netzwerkfähigen Polysaccharide liegt im Bereich von 50% - 97%, vorzugsweise im Bereich von 55% - 95%, noch bevorzugter im Bereich von 60% - 95%, am bevorzugtesten im Bereich von 70% - 95%.
2. Die Überhitzungs-Temperatur während der Lösung der vorliegenden Stärke beträgt mindestens 100°C, vorzugsweise mindestens 130°C, noch bevorzugter mindestens 160°C, insbesondere mindestens 180°C. Sie kann maximal bis 200°C betragen, wobei solch hohe Temperaturen jedoch nur für sehr kurze Zeiten zur Anwendung kommen können. Hohe Überhitzungs-Temperaturen wirken sich stabilisierend auf die Lösung aus, insbesondere das Retrogradationsverhalten betreffend.

Eine getrennte Aufbereitung von vorliegender Stärke und vorliegenden Polysacchariden ist insofern von Bedeutung, als die notwendigen Überhitzungs-Temperaturen für vorliegende Stärken tiefer liegen als für netzwerkfähige Polysaccharide, insbesondere für netzwerkfähige Stärke. Ausserdem werden die vorliegenden Stärken üblicherweise bei tieferen Weichmacher- oder Lösungsmittelgehalten gelöst als netzwerkfähige Polysaccharide. Würden die Komponenten zusammen gelöst, wird dadurch der für netzwerkfähige Polysaccharide zur Verfügung stehende Weichmacher- oder Lösungsmittelgehalt reduziert und dadurch die gewünschte Lösung erschwert. Durch ein individuelles Aufarbeiten können weiter die Lösungsparameter für die verschiedenen Komponenten optimal eingestellt werden.

### 2. Lösen und Kühlen netzwerkfähiger Polysaccharide

Erst durch geeignetes Lösen von netzwerkfähigen Polysacchariden wird ihr Potential zur Bildung von Netzwerken freigesetzt. Durch Plastifizierung, wie dies beispielsweise bei der Herstellung von Thermoplastischer Stärke üblich ist, ist dies höchstens teilweise gewährleistet oder sind bei tiefer Weichmacherkonzentration sehr hohe Temperaturen notwendig, die dann zu einem starken thermischen Abbau führen. Der Lösevorgang von netzwerkfähigen Polysacchariden wie auch von netzwerkfähigen Stärken ist ein vielstufiger und komplexer Vorgang. Das Lösen erstreckt sich üblicherweise über einen Temperaturbereich von einigen °C, wobei sukzessive Ordnungsstrukturen aufgelöst werden, bis eine vollständige Lösung erfolgt ist. Der Temperaturbereich zeigt ausserdem eine starke Abhängigkeit von der Konzentration. Der Lösevorgang ist weiterhin auch abhängig von einer mechanischen Beanspruchung durch Scherung, wodurch das Lösen bei tieferer Temperatur erfolgen kann, sowie vom Druck, der Lösungszeit, der Aufheizgeschwindigkeit und dem pH. Von entscheidender Bedeutung für die Erfindung ist eine Überhitzung der Lösung von netzwerkfähigem Polysaccharid, insbesondere von netzwerkfähiger Stärke, wodurch nach Abkühlen stabile Lösungen erhalten werden können, beziehungsweise die Gelierung zu tieferen Temperaturen hin verschoben werden kann. Unter Überhitzung wird der Vorgang verstanden, wobei eine Temperatur angewendet wird, die höher liegt als die minimale Lösungstemperatur. Die verschiedenen Parameter des Löse- und Abkühlungsvorgangs sind für die Struktur und Eigenschaften der nach dem Mischvorgang mit vorliegenden Polysacchariden sich ergebenen Gele von zentraler Bedeutung.

Eine weitere Möglichkeit zur Stabilisierung von Lösungen netzwerkfähiger Polysaccharide besteht im Einsatz von Keimstabilisatoren. Im Allgemeinen werden als Keimstabilisatoren stark verzweigte Polysaccharide verwendet. Sie verhindern im Temperaturbereich der für das Tauchverfahren verwendeten Lösung wie auch beim Kühlen der gelösten netzwerkfähigen Polysaccharide, insbesondere der netzwerkfähigen Stärke, das Weiterwachsen von Kristallit-Keimen und dadurch die bei diesen Temperaturen unerwünschte Retrogradation. Beispiele sind Glycogen, Amylopektin oder Agaropektin. Vorzugsweise werden Amylopektine mit einem Blue-Value von kleiner als 0.08 und/oder mit einer lod-Affinität von kleiner als 0.7g/100g eingesetzt.

Da netzwerkfähige Polysaccharide Lipide und Proteine enthalten können, welche mit den linearen Anteilen der netzwerkfähigen Polysaccharide Komplexe bilden und damit diese linearen Anteile für die Netzwerkbildung nicht mehr zur Verfügung stehen, ist es bei höheren Lipid- und Proteinanteilen angezeigt, diese Stoffe vorgängig durch Extraktion zu entfernen. Sie können jedoch auch nach dem Lösevorgang, bei der nachfolgenden Abkühlung, wobei sie aus der Lösung ausfallen, durch Filtration aus dem Prozess entfernt werden.

Die Parameter des Löse- und Kühlungsvorgangs sind wie folgt:
1. Der Weichmachergehalt WM_{d} in Gew.% der netzwerkfähigen Polysaccharide in Schritt d) und e) liegt im Bereich 50% - 99%, vorzugsweise im Bereich 60% - 97%, noch bevorzugter im Bereich 70% - 97, am bevorzugtesten im Bereich 80% - 97%. Hohe Weichmacher- oder Lösungsmittel-Gehalte sind von Bedeutung, um hohe Überhitzungstemperaturen bei geringem thermischem Abbau anwenden zu können, da die minimale Lösungstemperatur mit sinkendem Weichmachergehalt stark ansteigt.
2. Der Druck p während dem Überführen in Schritt d) und e) ist identisch mit dem Wasserdampfdruck p_{w}(T) bei der jeweiligen Temperatur, vorzugsweise maximal 2 p_{w}(T), noch bevorzugter maximal 5 p_{w}(T), insbesondere maximal 10 p_{W}(T), am bevorzugtesten maximal 100 p_{w}(T).
3. Die Überhitzungs-Temperatur T_{LÜ} in Schritt d) beträgt mindestens 100°C, vorzugsweise mindestens 130°C, noch bevorzugter mindestens 160°C, insbesondere mindestens 180°C, am bevorzugtesten mindestens 200°C. Sie kann maximal bis 260°C betragen, wobei solch hohe Temperaturen jedoch nur für sehr kurze Zeiten zur Anwendung kommen können. Hohe Temperaturen T_{LÜ} wirken sich stabilisierend auf die Lösung aus, d.h. je grösser T_{LÜ} - T_{L0}, bei umso tieferer Temperatur bleibt die Lösung danach noch stabil, wodurch der Verfahrensspielraum vergrössert und die Einstellung einer für das Tauchverfahren praktikablen Temperatur der Gesamtmischung ermöglicht wird.
4. Die Zeitdauer delta t_{d} des Überführens in Schritt d) beträgt maximal 7 min, vorzugsweise maximal 3min, noch bevorzugter maximal 1 min, insbesondere maximal 0.5min, am bevorzugtesten maximal 0.2min, minimal beträgt die Zeitdauer 5sec. Kurze Überführungszeiten sind insbesondere bei hohen Temperaturen T_{LÜ} zur Unterdrückung von thermischem Abbau von Bedeutung.
5. Die Aufheizgeschwindigkeit v_{d} beim Überführen in Schritt d) beträgt mindestens 1 °C/min, vorzugsweise mindestens 10°C/min, noch bevorzugter mindestens 50°C/min, insbesondere mindestens 100°C/min, am bevorzugtesten mindestens 200°C/min, maximal beträgt die Aufheizgeschwindigkeit etwa 300°C/min. Hohe Aufheizgeschwindigkeiten sind insbesondere bei hohen Konzentrationen C_{PN}, bei hohen Molekulargewichten dieser Polysaccharide, bei hoher Temperatur T_{LÜ} in Schritt d) und zur Unterdrückung eines thermischen Abbaus von netzwerkfähigen Polysacchariden wichtig.
6. Die Temperatur T_{L1} Schritt e) in beträgt maximal 0.9 T_{LÜ}, vorzugsweise maximal 130°C, noch bevorzugter maximal 100°C, insbesondere maximal 70°C, am bevorzugtesten maximal 50°C. Die minimale Temperatur liegt bei etwa 35°C.
7. Die Zeitdauer delta tₑ des Überführens in Schritt e) beträgt maximal 7min, vorzugsweise maximal 3min, noch bevorzugter maximal 1min, insbesondere maximal 0.5min, am bevorzugtesten maximal 0.2min, die kürzesten Zeiten liegen bei etwa 5sec.
8. Die Abkühlgeschwindigkeit vₑ beim Überführen in Schritt e) beträgt mindestens 5°C/min, vorzugsweise mindestens 30°C/min, noch bevorzugter mindestens 70°C/min, insbesondere mindestens 110°C/min, am bevorzugtesten mindestens 200°C/min, maximal beträgt die Abkühlungsgeschwindigkeit etwa 300°C/min.
9. Der pH in den Schritten d) und e) liegt für Stärke im Bereich 5 - 12, vorzugsweise im Bereich 6 -12, noch bevorzugter im Bereich 7 bis 12. Ein erhöhter pH erleichtert die Löslichkeit von netzwerkfähiger Stärke. Gegebenenfalls wird der pH der Gesamtmischung in Schritt g) durch Zugabe einer Säure oder Base auf den gewünschten Wert, vorzugsweise auf pH 6 - 8 eingestellt.
10. Die Schergeschwindigkeit G_{d} in den Schritten d) und/oder e) und f) beträgt mindestens 10/s, vorzugsweise mindestens 100/s, noch bevorzugter mindestens 1000/s, insbesondere mindestens 10'000/s, am bevorzugtesten mindestens 50'000/s. Maximale Schergeschwindigkeiten liegen bei rund 100'000/s. Durch hohe Schergeschwindigkeiten kann in Schritt d) die Löslichkeit insbesondere von netzwerkfähigen Polysacchariden mit hohem Molekulargewicht deutlich verbessert werden und können somit höhere Konzentrationen C_{PN} verarbeitet werden. Weiterhin wirken sich hohe Schergeschwindigkeiten wie auch hohe Überhitzungstemperaturen stabilisierend auf die Lösung aus.

Die verschiedenen Parameter des Lösungs- und Abkühlungsvorgangs werden entsprechend den vorstehenden Angaben dahingehend optimiert, um bei geringfügigem thermischem Abbau stabile Lösungen im Temperaturbereich von 40°C bis 70°C, vorzugsweise von 45°C bis 60°C zu erhalten. Dies ist eine Voraussetzung, um nach erfolgter Mischung mit vorliegenden Stärken Kapseln im Tauchverfahren unter konstanten Bedingungen durchführen zu können. Entsprechend den obigen Bedingungen 1 bis 10 behandelte gelöste netzwerkfähige Polysaccharide werden anschliessend mit vorliegender Stärke gemischt, um nach erfolgter Formgebung Netzwerke zu erhalten, wobei beim Einsatz von netzwerkfähiger Stärke als netzwerkfähiges Polysaccharid sowohl netzwerkfähige Stärke als auch vorliegende Stärke durch Heterokristallisation einen Beitrag zum entstehenden Netzwerk oder Gel leisten.

Nachdem ein netzwerkfähiges Polysaccharid oder eine Mischung von netzwerkfähigen Polysacchariden entsprechend obigen Bedingungen gelöst worden sind, können sie direkt mit der Lösung von vorliegender Stärke gemischt werden oder aber zwei oder mehrere Lösungen werden zuerst zusammengeführt, gemischt und dann vorliegender Stärke zugeführt. Es ist in bestimmten Fällen auch möglich, aufbereitete netzwerkfähige Polysaccharide in jeweils verschiedene erste Fluide oder Lösungen von vorliegenden Stärken einzumischen und diese Mischungen dann zu einer Gesamtmischung zu vereinigen.

### 3. Mischen von vorliegender Stärke mit netzwerkfähigen Polysacchariden

Insbesondere um einphasige Gele zu erhalten, ist eine molekulardisperse Mischung von vorliegenden Stärken und netzwerkfähigen Stärken oder Polysacchariden eine wesentliche Voraussetzung. Solche Mischungen können durch Anwendung von Scherung und hohen Schergeschwindigkeiten erhalten werden. Ist eine molekulardisperse oder nahezu molekulardisperse Mischung eingestellt worden, kann eine Phasenseparation durch die kinetische Kontrolle des Prozesses eingeschränkt oder ganz verhindert werden. Dies bedeutet eine entsprechende Kontrolle der Abkühlungsgeschwindigkeit, wobei der einphasige thermodynamisch metastabile Zustand eingefroren werden kann.
1. Der Weichmachergehalt WM₂ in Gew.% unmittelbar nach Schritt g) liegt im Bereich von 50% - 97%, vorzugsweise im Bereich von 55% - 95%, noch bevorzugter im Bereich von 60% - 95%, am bevorzugtesten im Bereich von 70% - 95%.
2. Die Schergeschwindigkeit G_{g} beim Mischen von erstem Fluid mit zweitem Fluid beträgt mindestens 10/s, vorzugsweise mindestens 100/s, noch bevorzugter mindestens 1000/s, insbesondere mindestens 1 0'000/s, am bevorzugtesten mindestens 50'000/s. Die maximale Schergeschwindigkeit liegt bei etwa 100'000/s. Durch hohe Schergeschwindigkeiten wird bevorzugt eine mokelulardisperse Mischung der Fluide erreicht, was für hohe resultierende Netzwerkdichten N₀/V₀ und insbesondere für einphasige Netzwerke eine Voraussetzung ist. Ausserdem wird durch hohe Schergeschwindigkeiten G_{g} eine grosse Zahl von möglichst kleinen die Netzwerkelemente bildenden Kristalliten erhalten, was sich vorteilhaft auf die mechanischen Eigenschaften der Formkörper auswirkt.
3. Die Konzentration C_{PNM} der entsprechend den Schritten d) bis f) verarbeiteten netzwerkfähigen Polysaccharide in der Mischung von Schritt g) in Gew.% beträgt 1% - 95%, vorzugsweise 2% - 70%, noch bevorzugter 3% bis 50%, insbesondere 4% bis 30%, am bevorzugtesten 5% - 25%.
4. Die getroffenen Verfahrensmassnahmen ermöglichen die Einstellung einer Temperatur der Lösung der Gesamtmischung (Tauchbadtemperatur) im Bereich von 40°C - 70°C, vorzugsweise im Bereich 45°C - 60°C. Eine möglichst tiefe Tauchbadtemperatur ist zur Verringerung von Abdampfverlusten von Weichmacher oder Lösungsmittel und damit für konstante Eigenschaften der Lösung von Bedeutung, für die industrielle Herstellung von Polysaccharid-Hartkapseln eine Voraussetzung.

Verschiedene netzwerkfähige Polysaccharide können zusammen gelöst, abgekühlt und dann vorliegender Stärke zugemischt werden. Da jedoch verschiedene netzwerkfähige Polysaccharide eine unterschiedliche Lösecharakteristik aufweisen, ist es meist sinnvoll, sie getrennt aufzubereiten und getrennt dem Mischvorgang zuzuführen.

Die Schritte a) bis j), werden vorzugsweise zumindest in Teilbereichen kontinuierlich durchgeführt, wobei die geeignete Verfahrenszone für die Schritte a) bis g) der Prozessraum mindestens eines Mischers ist und die Schritte a) bis g) kontinuierlich in aufeinanderfolgenden Abschnitten des mindestens einen Mischers und die Schritte h) bis j) in einer dem mindestens einen Mischer nachgeschalteten Formgebungs- und Konditionierungseinheit stattfinden. Der mindestens eine Mischer kann ein Einschnecken- oder ein Zweischnecken- oder ein Mehrschnecken- oder ein Ringextruder oder ein Kokneter oder ein statischer Mischer, ein Rührwerk, ein Ystral-Mischer, ein Jet-Cooker oder eine bezüglich Temperatur und Druck regelbare Verfahrensstrecke sein, die auch Scherung induzierende Mischelemente enthält. Die Formgebung ist grundsätzlich analog zur Herstellung von Hartkapseln im Tauchverfahren auf Basis von Gelatine, wobei mit einem Spezialfett beschichtete Formstifte in einem zeitlich optimierten Ablauf in ein Vorratsgefäss mit geschmolzener Gelatine eingetaucht, herausgezogen und rotiert werden, wonach sich der Gelatine-Film auf den Formstiften durch Gelierung verfestigt und anschliessend konditioniert, d.h. auf den gewünschten Endwassergehalt getrocknet werden kann. Nach erfolgter Konditionierung können die Formteile entformt werden. Anstelle der geschmolzenen Gelatine wird jedoch für die Herstellung von Hartkapseln auf Basis von Polysaccharid-Gel eine vorliegende Stärke, netzwerkfähiges Polysaccharid und Weichmacher beziehungsweise Lösungsmittel enthaltende viskose Lösung oder Schmelze verwendet. Entscheidend ist, dass deren Temperatur genügend hoch ist, damit im Vorratsgefäss keine Retrogradation oder Gelierung stattfinden kann und das Fluid zeitlich stabil bleibt. Andererseits darf die Temperatur nicht zu hoch sein, damit die Viskosität durch Verdampfungsverluste von Weichmacher mit der Zeit nicht ansteigt. Die Temperaturen des Fluids im Vorratsgefäss bewegen sich rezepturabhängig im Bereich von 40 - 70°C, geringe Verdampfungsverluste können wie bei Gelatine durch Zufuhr von Weichmacher, vorzugsweise von Wasser, kompensiert werden.

Die erhaltenen Polysaccharid-Hartkapseln können analog zu den Gelatine-Hartkapseln verwendet, d.h. mit denselben Wirkstoffen befüllt und eingesetzt werden. Stärke-Gel-Hartkapseln weisen infolge des kristallinen Anteils einen prebiotischen Effekt sowie einen gegenüber amorpher Stärke einen reduzierten Glyceamic-Index auf, wodurch gegenüber Gelatine weitere Vorteile entstehen. Ausserdem können die erfindungsgemässen stabilisierten und gelierfähigen Lösungen auch zur Beschichtung von Tabletten verwendet werden (Gel-Caplets). Die Gel-Beschichtung ermöglicht unter anderem einen guten Schutz des beschichteten Inhalts vor Sauerstoff (Barriere).

### Weichmacher beziehungsweise Lösungsmittel

Als Weichmacher können grundsätzlich dieselben Lösungsmittel, Weichmacher und Weichmachermischungen verwendet werden, die entsprechend dem Stand der Technik als Lösungsmittel, Weichmacher und Weichmachermischungen für die entsprechenden Polysaccharide und Hydrokolloid-Polysaccharide eingesetzt werden, bevorzugt werden sie aus folgender Gruppe ausgewählt:
➢ Wasser; Glycerin; Glycerinethoxylat; Polyglycerine; Di-, bis Decaglycerine; Polyglycerinmonoethoxylate; Reaktionsprodukte von Glucose mit Ethylenoxid; Glucosemonoethoxylat; Glucoside; Butylglucosid; Aphamethylglucosid; Maltose, Glucotri- und höhere Glucopolysaccharide, Mono- und Oligosaccharid-Sirupe; Alkohole; Polyalkohole; Butanol; Erythritol; Pentaerythritol; Triethylolpropan; Trimethylolpropan; Triethylpropanmonoethoxylat; Propandiole; Butandiole; Pentandiole; Hexandiole; Hexantriole; Polyvinylalkohole mit 3 bis 20 Monomereinheiten; ganz oder teilweise zu Polyvinylalkoholen hydrolysierte Polyvinylacetate; Trihydroxymethylaminomethane; Aminoalkohole; Fettalkohole; Amine; Hydroxyalkydamin; Ethylendiamin; Amide; Esteramide; Formamid; Säureamide; Sulfoxide; DMSO; quaternäre Ammonium-Verbindungen; Glycol, Ethylenglycol; Ethylendiglycol; Ethylentriglycol; Propylenglycol; Propylendiglycol; Propylentriglycol; Neopentylglycol; Polyethylenglykole; Polypropylenglycol; Polyglycole; Pyrrolidone; 2-Pyrrolidon oder 1-Methyl-2-Pyrrolidon; Caprolactan; Polycaprolactan; Sorbitol; Sorbitolacetat; Sorbitoldiacetat; Sorbitolmonoethoxylat; Sorbitoldipropoxylat; Sorbitoldiethoxylat; Sorbitolhexaethoxylat; Salze von Carboxymethylsorbitol; Aminosorbitol; Maltitol; Mannitol; Mannitolmonoacetat; Mannitolmonoethoxylat; Xylitol; Arabitol; Adonitol; Iditol; Galactitol; Allitol; Säuren; Carboxylsäuren; Ameisensäure; Essigsäure; Bernsteinsäure; Bernsteinsäureanhydrid; Adipinsäure; Milchsäure; Weinsäure; Citronensäure: Apfelsäure; Hydroxybuttersäure; Maleinsäure; Fettsäuren; Salze; Dimethylsulfoxid; Harnstoff; Ionen; Ionen der Alkali- und Erdalkalimetalle; chemisch veränderte, insbesondere durch Veresterung erhaltenen Elemente dieser Gruppe; Mischungen von Elementen dieser Gruppe.

Weichmacher, Weichmachermischungen oder Lösungsmittel werden üblicherweise vorliegender Stärke in Schritt b) und den netzwerkfähigen Polysacchariden in Schritt d) zugeführt. Zusätzlicher Weichmacher kann ausserdem auch in mindestens einem der Schritte a), c), e) f) oder g) dem Verfahren zugeführt werde.

### Zusätze

### 1. Keimstabilisatoren

Keimstabilisatoren können in einem der Schritte a) bis g) zugeführt werden, um die Lösung oder die Lösungen zu stabilisieren. Im Allgemeinen werden als Keimstabilisatoren stark verzweigte Polysaccharide verwendet, welche keine Gelbildung zeigen oder erst nach Tagen oder Wochen nur sehr schwache Gele bilden. Beispiele sind Glycogen, Amylopektin oder Agaropektin. Vorzugsweise werden Amylopektine mit einem Blue-Value von kleiner als 0.08 und/oder mit einer lod-Affinität von kleiner als 0.7g/100g eingesetzt.

### 2. Additive

Additive können in mindestens einem der Schritte a) bis g) zur Verbesserung der Verarbeitbarkeit, zur Beeinflussung der Netzwerkbildung und zur Modifikation der Produkteigenschaften mit Anteilen in Gew.% von 0.01 % bis 10%, vorzugsweise von 0.02% bis 7%, noch bevorzugter von 0.03% bis 5% zugeführt werden. Unter anderem können Additive und Hilfsstoffe, welche bei der Herstellung von Gelatine-Kapseln dem Stand der Technik entsprechen, auch für Polysaccharid-Netzwerke eingesetzt werden. Additive werden insbesondere aus folgender Gruppe von Stoffen ausgewählt:
➢ Lebensmitteladditive, insbesondere Antioxidantien und Lebensmittel-Stabilisatoren; Glycerinderivate; Mono-, Di- und Triglyceride und deren Stearate; Glycerinmonostearat; Mono-, Di-, oder Triglyceride von Speisefettsäuren; Polyglycerinester, insbesondere der Speisefettsäuren; Polyethylenglycole; Polyethylenglycolester, insbesondere der Speisefettsäuren; Lecithine; nichtionische und ionische Netzmittel und Tenside; Emulgatoren; Komplexbildner; Amylose-Komplexbildner; Na-2-stearoyl-Lactat; aliphatische Alkohole; Fettsäuren, insbesondere Stearinsäuren, aliphatische und aromatische Ester; Pyridin; Zucker; Zuckerester, insbesondere Zuckerester der Sepeisefettsäuren; Fette; Fettsäurenester; Wachse, insbesondere vegetarische Wachse wie Carnauba-Wachs, Candelillawachse, Japanwachs, Ouricurywachs, Gagelstrauchwachs, Jojobawachs; Polyolefinwachse; Naturharze; Schellack; Chitin; Kollagen; Casein; Mono- und Oligosaccharide; Dextrane; Proteine; Peptide; Polypeptide, insbesondere pflanzliche Polypeptide; Cellulose, Cellulosederivate, insbesondere hydroxypropylierte Cellulose; Hydrocolloide, insbesondere Alginate, Carrageenane, Galactomannane, Glucomannane; Farbstoffe; als Lebensmittel verwendbare Stoffe; Aromastoffe; Mischungen von Elementen dieser Gruppe.

### 3. Füllstoffe

Füllstoffe können in mindestens einem der Schritte a) bis g), zugeführt werden, um Eigenschaften des Werkstoffs zu modifizieren und/oder die spezifischen Rohstoffkosten per Kilogramm zu reduzieren. Sie werden sie werden aus folgender Gruppe ausgewählt:
➢ Mineralien, insbesondere Titandioxid; Lignin; Fasern, insbesondere Naturfasern wie Baumwolle, Hanffasern, Flachs, Ramie, Jutefasern; native Stärke; inhibierte Stärke; vernetzte Stärke; Stärke mit einem Amylose-Gehalt von mehr als 40%; Mischungen von Elementen dieser Gruppe.

### 4. Sprengmittel

Als Sprengmittel können entsprechend dem Stand der Technik in der Galenik verwendete Stoffe verwendet werden, wie beispielsweise Carbonate und Hydrogencarbonate der Alkali- und Erdalkaliionen, insbesondere Calciumcarbonat. Ausserdem kommen auch Amylasen in Frage. Ein Sprengmittel oder Mischungen von Sprengmitteln können in einem der Schritte a) bis c) oder g) zudosiert werden. Sie werden beim Einsatz hoher Anteile von netzwerkfähigen Polysacchariden verwendet, um den Zerfall der Kapseln nach der Verabreichung zu beschleunigen. Bei niederen Anteilen von netzwerkfähigen Polysacchariden werden sie nicht benötigt.

### Strukturtyp von Stärke-Netzwerken

Durch geeignete Prozessführung kann erreicht werden, dass die sich bildenden Kristallite bei Raumtemperatur bevorzugt eine A-Struktur aufweisen. Dieser Strukturtyp zeigt gegenüber dem bei Raumtemperatur stabilen B-Strukturtyp bei gleicher Luftfeuchtigkeit eine massiv reduzierte Wasseraufnahme, wodurch sich ein günstigeres Sorptionsverhalten ergibt. Der bei Raumtemperatur metastabile A-Strukturtyp kann durch kinetische Kontrolle eingefroren und so auch bei Raumtemperatur erhalten werden. Eine weitere Möglichkeit zur Einstellung des A-Strukturtyps ist gegeben durch eine Wärmebehandlung, wobei der B-Strukturtyp in den gewünschten A-Strukturtyp umgewandelt wird. Die notwendige Temperatur, die nur kurzzeitig angewendet werden muss, liegt oberhalb von 100°C.

### Parameter

- T_{L0}: Minimale Temperatur, bei der sich die netzwerkfähigen Polysaccharide lösen
- T_{LR}: Temperatur der Rekristallisation (oder Retrogradation) der netzwerkfähigen Polysaccharide im thermodynamischen Gleichgewicht nach dem Lösen bei T_{L0}
- T_{LÜ}: Überhitzungstemperatur T_{LÜ} > T_{L0}
- T_{LM}: Temperatur bei der der metastabile Zustand des unterdrückten Keimwachstums für 10 Sekunden aufrechterhalten werden kann
- deltaT_{LU}: Unterkühlung delta T_{LU} = T_{LR} - T_{LM}
- T_{L1}: Temperatur der Lösung beim Einmischen des zweiten oder dritten Fluids in das erste Fluid, T_{LM} < T_{L1} < T_{LÜ}, insbesondere T_{LM} < =T_{L1} < T_{LR}
- T₁: Temperatur des ersten Fluids vor der Zuführung des zweiten oder dritten Fluids
- T_{M}: Temperatur während des Mischvorgangs
- T₃: Temperatur am Ende des Mischvorgangs
- T_{K}: Temperatur zu Beginn der Netzwerkbildung
- delta t_{d}: Zeitdauer des Ueberführens in Schritt d)
- delta tₑ: Zeitdauer des Ueberführens in Schritt e)
- v_{d}: Aufheizgeschwindigkeit in Schritt d)
- vₑ: Abkühlgeschwindigkeit in Schritt e)
- C_{PN}: Konzentration der netzwerkfähigen Polysaccharide im zweiten oder dritten Fluid
- C_{PNM}: Konzentration der netzwerkfähigen Polysaccharide in der Mischung
- C_{Sta}: Konzentration des Keimstabilisators im ersten Fluid
- C_{StaM}: Konzentration des Keimstabilisators in der Gesamtmischung
- WM_{d}: Weichmachergehalt in Schritt d)
- WM₁: Weichmachergehalt der vorliegenden Stärke
- WM₂: Weichmachergehalt der Gesamtmischung nach Zugabe des zweiten oder dritten Fluids
- WM₃: Weichmachergehalt am Ende des Mischvorgangs
- WM₀: Weichmachergehalt bei der Netzwerkbildung
- W₀: Wassergehalt bei der Netzwerkbildung
- W₁: Wassergehalt nach Quellung eines Films mit W₀ in Wasser
- G_{d}: Schergeschwindigkeit in Schritt d)
- G_{g}: Schergeschwindigkeit in Schritt g)
- p_{w}(T): Wasserdampfdruck bei der Temperatur T
- N₀/V₀: Netzwerkdichte nach erfolgter Netzwerkbildung
- DP: Mittlerer Polymerisationsgrad
- CL: Mittlere Kettenlänge (Anzahl Monomereinheiten unverzweigter Kettensegmente)
- Q_{b}: Durchschnittlicher Verzweigungsgrad: Anzahl Mole der verzweigten Monomereinheiten/Anzahl Mole der gesamten Monomereinheiten (für Stärke: Anzahl Mole der verzweigten alpha-Glucan-Einheiten/Anzahl Mole der gesamten alphaGlucan-Einheiten)
- BV: Blue-Value
- IA: lod-Affinität [g/100g]
- M_{w}: Gewichtsmittel der Molekulargewichtsverteilung

Die Weichmacher- und Wassergehalte beziehen sich jeweils auf vorliegende und netzwerkfähige Polysaccharide, d.h. auf Polysaccharide, welche konstituierende Bestandteile des Netzwerks sind. Ein Netzwerk enthaltend beispielsweise 10g vorliegende Stärke, 3g netzwerkfähige Stärke, 11 g Wasser, 2g Glycerin, 7g Zucker und 5g eines Zusatzes weist daher einen Weichmachergehalt WM₀ von 100*(11+2)/(11+2+10+3) = 50% und einen Wassergehalt von 100*11/(11+10+3) = 45.8% auf.

### Eigenschaften der Weichkapseln

Der Quellgrad Q (Q = Volumen nach der Quellung/Volumen vor der Quellung) der bei 50% Luftfeuchtigkeit und 25°C konditionierten Kapselhülle beim Einlegen in Wasser bei 25°C im maximal gequollenen Zustand liegt im Bereich 1.1 - 20, vorzugsweise im Bereich 1.1 - 10, am bevorzugtesten im Bereich 1.1 - 7.

Die Bruchfestigkeit der bei 50% Luftfeuchtigkeit und 25°C konditionierten Kapselhüllen liegt im Bereich 1 MPa - 30MPa, vorzugsweise im Bereich 1.5MPa - 20MPa, am bevorzugtesten im Bereich 2MPa bis 17MPa.

Die Bruchdehnung der bei 50% Luftfeuchtigkeit und 25°C konditionierten Weichkapselhülle liegt im Bereich 10% - 100%, vorzugsweise im Bereich 15% bis 75%, am bevorzugtesten im Bereich 20% - 50%

Der Gesamtweichmachergehalt der Kapselhüllen in Gew.% nach Konditionierung bei 50% Luftfeuchtigkeit und 25°C liegt im Bereich 10% - 70%, vorzugsweise im Bereich 14% - 60%, am bevorzugtesten im Bereich 18% - 50%.

### Beispiele

Weitere Vorteile, Merkmale und Anwendungsmöglichkeiten der Erfindung ergeben sich aus der folgenden Beschreibung von nicht einschränkend aufzufassenden Ausführungsbeispielen.

### Beispiel 1

### Rezeptur

79.3% (bezogen auf das Trockengewicht) vorliegende Stärke: 70% Kartoffelstärke (10% Wasser), nativ, 30% Waxy-Mais (10% Wasser), nativ
20% (bezogen auf das Trockengewicht) netzwerkfähige Stärke: 30% hochamylosehaltige Mais-Stärke, nativ (50% Amylosegehalt, 15% Wasser), 40% enzymatisch entzweigte Tapioka Stärke (15% Wasser), 30% Maltodextrin (15% Wasser)
Zusatz: 0.7% Lecithin

Weichmacher in Schritt b): 65% Wasser, 8% Sorbitol, 6% Maltitol (bezogen auf die vorliegenden Stärken, Trockengewicht)
Weichmacher in Schritt d): 85% Wasser, 5% Glycerin (bezogen auf die netzwerkfähigen Stärken, Trockengewicht)

### Verfahren

Lösen von vorliegenden Stärken bei 130°C unter Scherung (Ystral-Mischer), anschliessend Kühlung auf 50°C. Lösen von netzwerkfähigen Stärken bei 190°C (1min) unter Scherung (Ystral-Mischer), anschliessend Kühlung innerhalb von 2min auf 50°C und Herstellung der Gesmtmischung bei dieser Temperatur mittels eines Ystral Mischers. Tauchverfahren bei 50°C.

### Beispiel 2

### Rezeptur

82% (bezogen auf das Trockengewicht) vorliegende Stärke: 60% Kartoffelstärke (10% Wasser), nativ, 40% Tapioka (10% Wasser), nativ
18% (bezogen auf das Trockengewicht) netzwerkfähige Stärke: 40% entzweigte hochamylosehaltige Mais-Stärke (15% Wasser), 40% enzymatisch entzweigte Tapioka Stärke (15% Wasser), 20% Maltodextrin (15% Wasser)

### Verfahren

Lösen von vorliegenden Stärken bei 130°C unter Scherung (Sulzer-Mischer), anschliessend Kühlung auf 55°C. Lösen von netzwerkfähigen Stärken bei 185°C (1min) unter Scherung (Ystral-Mischer), anschliessend Kühlung innerhalb von 2min auf 55°C und Herstellung der Gesmtmischung bei dieser Temperatur mittels eines Rührwerks. Tauchverfahren bei 55°C.

### Beispiel 3

### Rezeptur

75% (bezogen auf das Trockengewicht) vorliegende Stärke: 60% Kartoffelstärke (10% Wasser), nativ, 40% Tapioka (10% Wasser), nativ
25% (bezogen auf das Trockengewicht) iota-Carrageenan (10% Wasser)

### Verfahren

Lösen von vorliegenden Stärken bei 130°C unter Scherung (Sulzer-Mischer), anschliessend Kühlung auf 55°C. Lösen von iota-Carrageenan 145°C (1 min) unter Scherung (Ystral-Mischer) in Gegenwart von Ca-lonen, anschliessend Kühlung innerhalb von 2min auf 55°C und Herstellung der Gesmtmischung bei dieser Temperatur mittels eines Rührwerks. Tauchverfahren bei 55°C.

## Patentansprüche

1. Verfahren zum Herstellen von Formkörpern auf Basis von Stärke-Gel im Tauchverfahren, **dadurch gekennzeichnet, dass** das Gel aus einer durch molekulardisperses Mischen hergestellten Gesamtmischung von mindestens einer Lösung von vorliegender Stärke sowie mindestens einer Lösung von netzwerkfähiger Stärke gebildet wird, worin die netzwerkfähige Stärke einen Verzweigungsgrad (Q_{b}) von 0.02 - 0.001 aufweist, wobei der mittlere Polymerisationsgrad DP > 100 ist und dass die Lösungen getrennt und individuell vorbereitet werden, wobei die Lösung von netzwerkfähiger Stärke überhitzt und **dadurch** stabilisiert wird.

2. Verfahren nach Anspruch 1, **dadurch gekennzeichnet, dass** die Temperatur der Gesamtmischung während dem Tauchverfahren bei einer Temperatur im Bereich von 40°C - 70°C gehalten wird, ohne dass dabei eine Retrogradation eintritt.

3. Verfahren nach einem der vorangehenden Ansprüche, **dadurch gekennzeichnet, dass** der Lösevorgang von netzwerkfähiger Stärke und/oder von vorliegender Stärke und/oder der Mischvorgang zur Herstellung der Gesamtmischung in Gegenwart von Scherung geschieht, wobei die Schergeschwindigkeit mindestens 10/s beträgt.

4. Verfahren nach einem der vorangehenden Ansprüche, **dadurch gekennzeichnet, dass** das Verfahren folgende Schritte in mindestens einer Verfahrenszone aufweist:
a) Zuführen jeweils einer vorliegenden Stärke;
b) Einwirken jeweils eines ersten Weichmachers auf die jeweils eine vorliegende Stärke;
c) Überführen der jeweils einen vorliegenden Stärke in jeweils ein erstes Fluid, wobei jeweils eine erste Lösung gebildet wird;
d) Überführen jeweils einer netzwerkfähigen Stärke in jeweils ein zweites Fluid durch Einwirken jeweils eines zweiten Weichmachers, wodurch jeweils eine zweite Lösung gebildet wird;
e) Überführen des jeweiligen zweiten Fluids in ein jeweiliges drittes Fluid, wodurch jeweils eine dritte Lösung gebildet wird;
f) Einführen des jeweiligen zweiten Fluids aus Schritt d) oder des jeweiligen dritten Fluids aus Schritt e) in eine der jeweiligen ersten Lösungen aus den Schritten a) bis c);
g) Vereinigen der jeweiligen Mischungen aus den Schritten a) bis f) zu einer Gesamtmischung;
h) Eintauchen einer Negativ-Form in die Gesamtmischung und Herausziehen der mit einem Film der Gesamtmischung beschichteten Negativ-Form;
i) Beginn der Bildung eines Stärke-Netzwerks oder Gels in dem in Schritt h) gebildetem Film der Gesamtmischung, insbesondere beim Einsatz von netzwerkfähiger Stärke durch Homokristallisation zwischen jeweiligen Makromolekülen und jeweiligen Makromolekülen der jeweils mindestens einen vorliegenden Stärke, nachdem die Schritte a) bis h) vollzogen sind;
j) Zuführen der in Schritt h) beschichteten Negativ-Form in eine Konditionierungsanlage und Trocknung der Formteile bei bereitgestelltem zeitlichem Temperatur-, Luftstrom- und Luftfeuchtigkeitsverlauf, wonach der verfestigte Film von der Negativ-Form entformt wird.

5. Verfahren nach einem der vorangehenden Ansprüche, **dadurch gekennzeichnet, dass** die Lösung von netzwerkfähiger Stärke und/oder die Gesamtmischung einen Keimstabilisator enthält.

6. Verfahren nach einem der vorangehenden Ansprüche, **dadurch gekennzeichnet, dass** in mindestens einem der Schritte a) bis g) mindestens ein Zusatz zugegeben wird.

7. Hartkapsel, **dadurch gekennzeichnet, dass** sie durch ein Verfahren gemäss einem der vorangehenden Ansprüche 1-6 hergestellt wird.

8. Hartkapsel nach Anspruch 7, **dadurch gekennzeichnet, dass** sie aus einer Lösung enthaltend vorliegende Stärke und netzwerkfähige Stärke durch einen Gelierungsvorgang hergestellt wird, wobei insbesondere eine Heterokristallisation an dem Gelierungsvorgang beteiligt ist und/oder das Stärke-Gel transparent ist.

9. Hartkapsel nach Anspruch 8, **dadurch gekennzeichnet, dass** die vorliegende Stärke mindestens eine Komponente von nicht-modifizierter vorliegender Stärke aufweist, insbesondere nur Komponenten von nicht-modifizierter Stärke enthält.

10. Hartkapsel nach einem der vorangehenden Ansprüche 7 bis 9, **dadurch gekennzeichnet, dass**, die Kapselhülle in Form eines Films von max. 0.3 mm Dicke, nachdem dieser Film getrocknet worden ist und in diesem Zustand in eine Atmosphäre mit maximal 43% Luftfeuchtigkeit bei Raumtemperatur gebracht und dort so lange aufbewahrt wurde, bis das Gewicht des Films sich nicht mehr änderte und der Film einen Wassergehalt von W₄₃ aufwies und, nachdem der Film darauf in eine Atmosphäre mit mindestens 90% Luftfeuchtigkeit bei Raumtemperatur gebracht wurde, bis das Gewicht sich nicht mehr änderte und der Film einen Wassergehalt von W₉₀ angenommen hat, die Differenz der Wassergehalte W₉₀ - W₄₃ in Gew.% 3% - 25% beträgt.

11. Hartkapsel nach einem der vorangehenden Ansprüche 7 bis 10, **dadurch gekennzeichnet, dass** der kristalline Anteil der Hartkapselhülle, erhalten durch Separation des kristallinen Anteils vom amorphen Anteil der Weitwinkel-Röntgendiffraktionskurve einer Probe mit 7 bis 14 Gew.% Wasser, zwischen den Streuwinkeln 3° < 2theta< 37°, 15% - 100%beträgt.

12. Hartkapsel nach einem der vorangehenden Ansprüche 7 bis 11, **dadurch gekennzeichnet, dass** die Kapselhülle aus transparentem Stärke-Gel besteht und/oder den röntgenographischen A-Strukturtyp aufweist.

13. Hartkapsel nach einem der vorangehenden Ansprüche 7 bis 12, **dadurch gekennzeichnet, dass** die Kapselhülle prebiotisch wirkt und/oder gegenüber thermoplastischer Stärke bei vergleichbarem Weichmachergehalt einen um 10% - 95 geringeren Glyceamic-Index aufweist.

## Claims

1. Method for the production of mouldings based on starch gel, by the immersion method, **characterized in that** the gel is formed from a total mixture of at least one solution of available starch and at least one solution of starch having network capabilities, which total mixture is prepared by molecular disperse mixing, wherein the starch having network capabilities has a degree of branching (Q_{b}) of less than 0.02 - 0.001, the average degree of polymerisation DP being >100, and **in that** the solutions are prepared separately and individually, the solution of starch having network capabilities being superheated and stabilised thereby.

2. Method according to Claim 1, **characterized in that** the temperature of the total mixture during the immersion method is kept at a temperature in the range of 40°C - 70°C without retrogradation occurring.

3. Method according to either of the preceding claims, **characterized in that** the dissolution process of starch having network capabilities and/or of available starch and/or the mixing process for the preparation of the total mixture takes place in the presence of shearing, the shear rate being at least 10/s.

4. Method according to any of the preceding claims, **characterized in that** the method comprises the following steps in at least one zone of the method:
a) addition of an available starch in each case;
b) action of a first plasticizer in each case on the one available starch in each case;
c) transfer of the one available starch in each case into a first fluid in each case, a first solution being formed in each case;
d) transfer of a starch having network capabilities in each case into a second fluid in each case by the action of a second plasticizer in each case, with the result that a second solution is formed in each case;
e) transfer of the respective second fluid into a respective third fluid, with the result that a third solution is formed in each case;
f) introduction of the respective second fluid from step d) or the respective third fluid from step e) into one of the respective first solutions from the steps a) to c);
g) combination of the respective mixtures from the steps a) to f) to give a total mixture;
h) immersion of a negative mould into the total mixture and withdrawal of the negative mould coated with a film of the total mixture;
i) beginning of the formation of a starch network or gel in the film of the total mixture which is formed in step h), in particular, with the use of starch having network capabilities, by homocrystallization between respective macromolecules and respective macromolecules of the at least one available starch in each case, after the steps a) to h) have been completed;
j) feeding of the negative mould coated in step h) into a conditioning unit and drying of the shaped articles with provided temperature, airflow and relative humidity curve as a function of time, after which the solidified film is removed from the negative mould.

5. Method according to any of the preceding claims, **characterized in that** the solution of starch having network capabilities and/or the total mixture contains a nucleus stabiliser,

6. Method according to any of the preceding claims, **characterized in that** at least one additive is added in at least one of the steps a) to g).

7. Hard capsule, **characterized in that** it is produced by a method according to any of the preceding Claims 1 - 6.

8. Hard capsule according to Claim 7, **characterized in that** it is produced from a solution containing available starch and starch having network capabilities by a gelling process, in particular heterocrystallization being involved in the gelling process and/or the starch gel being transparent.

9. Hard capsule according to Claim 8, **characterized in that** the available starch has at least one component of unmodified available starch, in particular contains only components of unmodified starch.

10. Hard capsule according to any of the preceding Claims 7 to 9, **characterized in that**, if the capsule shell is in the form of a film of not more than 0.3 mm thickness, and after this film has been dried and was brought in this state into an atmosphere having a maximum relative humidity of 43% at room temperature and was stored there until the weight of the film no longer changed and the film had a water content of W₄₃, and after the film was then brought into an atmosphere having a relative humidity of 90% at room temperature until the weight no longer changed and the film has assumed a water content of W₉₀, the difference between the water contents W₉₀ - W₄₃ in percent by weight is 3% - 25%.

11. Hard capsule, according to any of the preceding Claims 7 to 10, **characterized in that** the crystalline fraction, obtained by separation of the crystalline fraction from the amorphous fraction of the wide-angle X-ray diffraction curve of a sample with 7 to 14% by weight of water, between the scattering angles 3° < 2 theta < 37°, is 15% - 100%.

12. Hard capsule according to any of the preceding Claims 7 to 11, **characterized in that** the capsule shell consists of transparent starch gel and/or has the A structure type determined by X-ray diffraction.

13. Hard capsule according to any of the preceding Claims 7 to 12, **characterized in that** the capsule shell has a prebiotic effect and/or has a glycemic index which is 10% - 95% lower compared with thermoplastic starch at a comparable plasticizer content.

## Revendications

1. Procédé de fabrication d'objets moulés à base de gel d'amidon par immersion, qui se **caractérise en ce que** le gel d'amidon est formé à partir d'un mélange global par dispersion moléculaire d'au moins une solution d'un amidon de base et d'au moins un amidon capable de se réticuler, dans lequel l'amidon capable de se réticuler présente un degré de ramification (Q_{b}) de 0.02 - 0.001 et où le degré moyen de polymérisation est DP > 100, et où les solutions sont préparées séparément et individuellement en surchauffant la solution d'amidon pouvant se réticuler pour la stabiliser.

2. Procédé selon la revendication 1, se distinguant en ce que la température du mélange global est maintenue entre 40°C - 70°C pendant le procédé d'immersion, sans qu'il se produise une rétrogradation.

3. Procédé selon l'une des revendications précitées, **caractérisé en ce que** le processus de solution d'un amidon capable de se réticuler et/ou d'un amidon de base et/ou le processus du mélange pour réaliser le mélange global se fait en présence de cisaillement dont la vitesse comporte au moins 10/s.

4. Procédé selon l'une des revendications précitées, **caractérisé en ce que** le procédé s'effectue selon les étapes suivantes dans au moins une zone de procédure :
a) Ajouter à chaque fois un amidon de base ;
b) Action chimique d'un premier plastifiant sur l'amidon de base,
c) Transférer un amidon de base dans un premier fluide, pour former une première solution ;
d) Transférer un amidon capable de se réticuler dans un deuxième fluide sous l'action d'un deuxième plastifiant ce qui permet de réaliser une 2e solution.
e) Transférer le deuxième fluide dans un troisième fluide ce qui permet de réaliser une 3e solution
f) Introduire le deuxième fluide de l'étape d) et/ou du troisième fluide de l'étape e) dans l'un des premiers mélanges des étapes a) à c);
g) Réunir les mélanges des étapes a) à f) en un mélange global
h) Plonger une forme négative dans le mélange global et ensuite retirer la formée négative recouverte d'une couche du mélange global.
i) Début de la formation d'un réseau d'amidon ou d'un gel à partir du film du mélange global réalisé à l'étape h), en particulier en utilisant un amidon capable de se réticuler par homocristallisation entre ces macromolécules et les macromolécules d'un amidon de base, une fois les étapes a) à h) exécutées.
j) Introduire la forme négative revêtue d'une couche lors de l'étape h) dans un dispositif de conditionnement et puis séchage des parties moulées en réglant le temps de la température, du flux d'air et de l'hygrométrie avant de démouler la forme négative solidifiée.

5. Procédé selon l'une des revendications précédentes, **caractérisé en ce que** la solution d'amidon pouvant se réticuler et/ou le mélange contient un stabilisateur de germes.

6. Procédé selon l'une des revendications précédentes, **caractérisé en ce que** dans l'une au moins des étapes a) à g), on ajoute au moins un additif.

7. Capsule dure **caractérisée par le fait qu'**elle est réalisée selon un procédé répondant à l'une des revendications précitées 1 - 6.

8. Capsule dure selon la revendication 7, **caractérisée en ce qu'**elle est réalisée à l'aide d'un processus de gélification à partir d'une solution contenant un amidon de base et un amidon capable de se réticuler, où en particulier une hétérocristallisation participe au processus de gélification et que le gel d'amidon est transparent.

9. Capsule dure selon la revendication 8, **caractérisée en ce que** l'amidon de base contient au moins un composant d'amidon de base non modifié, en particulier contient seulement des composants d'amidon non modifié.

10. Capsules dures selon l'une des revendications 7 à 9, **caractérisées en ce que** l'enveloppe de la capsule formant un film épais de 0.3mm au minimum, que, une fois ce film séché et mis sous cet état dans une atmosphère comportant au maximum 43% d'humidité à la température ambiante, qu'il y soit conservé ainsi aussi longtemps jusqu'à ce que le poids du film ne change plus et que le film présente une teneur en eau de W₄₃ et que, une fois que le film mis ensuite dans une atmosphère avec au moins 90% d'humidité à la température ambiante, son poids ne change plus et ait pris une teneur en eau de W₉₀, la différence des teneurs en eau W₉₀ - W₉₀ se situant en poids % entre 3% - 25%.

11. Capsule dure selon l'une des revendications 7 à 10, **caractérisée en ce que** la part cristalline de l'enveloppe de la capsule dure, obtenue par séparation de la part cristalline de la part amorphe de la courbe de diffraction radiographique de grand angulaire d'une épreuve/échantillon avec un poids % en eau de 7 à 14, se situe entre les angles de dispersion 3° < 2δ < 37°, 15% - 100%.

12. Capsule dure selon l'une des revendications 7 à 11, **caractérisée en ce que** l'enveloppe de la capsule dure est réalisée à partir de gels d'amidon transparents, et/ou présente un type de structure A radiographique.

13. Capsule dure selon l'une des revendications précitées 7 à 12, **caractérisée en ce que** l'enveloppe de la capsule dure dispose d'un effet prébiotique et/ou présente par rapport à un amidon thermoplastique avec une teneur en plastifiant comparable un indice glycémique plus réduit aux environs de 10% - 95%.
